(19) **Europäisches Patentamt / European Patent Office / Office européen des brevets**

(11) **EP 4 778 629 A1**

(12) **EUROPEAN PATENT APPLICATION**
published in accordance with Art. 153(4) EPC

(43) Date of publication:
**22.07.2026 Bulletin 2026/30**

(21) Application number: **24865915.3**

(22) Date of filing: **13.09.2024**

(51) International Patent Classification (IPC):
**B01J 35/30** (2024.01)     **B01J 35/40** (2024.01)
**B01J 23/745** (2006.01)     **C07C 1/12** (2006.01)
**C10G 2/00** (2006.01)

(52) Cooperative Patent Classification (CPC):
**C10G 2/332; B01J 23/745; B01J 23/78;**
**B01J 35/30; B01J 35/40; B01J 35/50;**
**B01J 37/0201; B01J 37/0209; B01J 37/0238;**
**B01J 37/031; C10G 2/50; C10K 3/026;**
B01J 23/881; B01J 35/45; B01J 2235/30

(86) International application number:
**PCT/KR2024/014045**

(87) International publication number:
**WO 2025/058476 (20.03.2025 Gazette 2025/12)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **15.09.2023  KR 20230123492**

(71) Applicant: **LG Chem, Ltd.**
**Seoul 07336 (KR)**

(72) Inventors:
 • **PARK, Seung Won**
  **Daejeon 34122 (KR)**
 • **KIM, Won Hee**
  **Daejeon 34122 (KR)**

 • **JUNG, Da Won**
  **Daejeon 34122 (KR)**
 • **KIM, Seong Min**
  **Daejeon 34122 (KR)**
 • **KIM, Eui Tae**
  **Daejeon 34122 (KR)**
 • **MOON, Ji Won**
  **Daejeon 34122 (KR)**
 • **LEE, Yong Hee**
  **Daejeon 34122 (KR)**
 • **KIM, Ki Hwan**
  **Daejeon 34122 (KR)**

(74) Representative: **Hoffmann Eitle**
**Patent- und Rechtsanwälte PartmbB**
**Arabellastraße 30**
**81925 München (DE)**

(54) **CATALYST FOR CARBON DIOXIDE CONVERSION AND PREPARATION METHOD THEREFOR**

(57)    The present invention relates to a catalyst in the form of a core-shell, wherein the core is in the form of a secondary particle formed by agglomerating a plurality of primary particles, and the shell is formed by agglomerating a plurality of primary particles in the form of a core-shell including a primary particle and a shell layer surrounding the primary particle, and wherein the average diameter (R) of the core and the average thickness (L) of the shell formed by agglomerating a plurality of primary particles in the form of a core-shell satisfy Equation 1. When the catalyst of the present invention is used in a carbon dioxide conversion reaction, the conversion rate of carbon dioxide and the selectivity for hydrocarbons with 5 or more carbon atoms, which are useful components, is high.

EP 4 778 629 A1

FIG. 1

Fe-Al

Fe

R(μm)

Secondary Particle

L(μm)

Magnification of secondary particle

r(nm)

FeSi

l(nm)

Al

Primary Particle

**Description**

**[Technical Field]**

**CROSS-REFERENCE TO RELATED APPLICATION**

This application claims the benefit of priority to Korean Patent Application No. 10-2023-0123492 filed on September 15, 2023, the disclosure of which is incorporated herein by reference in its entirety.

**Technical Field**

The present invention relates to a novel carbon dioxide conversion catalyst capable of increasing the conversion rate of carbon dioxide and the selectivity for hydrocarbons having 5 or more carbon atoms in a reaction of converting carbon dioxide into hydrocarbons, while maintaining catalytic activity for a long period of time by minimizing sintering and coking phenomena that may occur during the reaction process.

**[Background Art]**

Carbon dioxide accounts for a large proportion of greenhouse gases, and thus, technologies for reducing the amount of carbon dioxide in the atmosphere are being studied in various ways. Typically, carbon capture and storage (CCS) technologies that capture and store carbon dioxide, and carbon capture and utilization (CCU) technologies that capture carbon dioxide and then utilize it in other fields are being studied in various ways.

Among the two categories of carbon dioxide treatment technologies, a representative technology among the CCU technologies that can utilize captured carbon dioxide beyond simple storage is a technology for producing economically valuable hydrocarbons from the captured carbon dioxide. Methods for producing hydrocarbons from carbon dioxide include electrochemical, photochemical, and thermochemical methods. However, the electrochemical and photochemical methods are difficult to produce long-chain hydrocarbons and have low conversion rates of carbon dioxide, so more in-depth research is needed until they can be applied to actual industrial fields. On the other hand, the thermochemical method is a method for producing various hydrocarbon gases from carbon dioxide using various catalysts at high temperatures and under a hydrogen atmosphere, and is currently the most widely used method because the conversion rate of carbon dioxide is high compared to the two methods described above.

It is known that a reaction for producing hydrocarbons from carbon dioxide is generally composed of two consecutive reactions. The first reaction is a reaction in which carbon dioxide is converted into carbon monoxide through a reverse water gas shift (RWGS) reaction, and the second reaction is a reaction in which the carbon monoxide produced through the first reaction is converted into hydrocarbons through a Fischer-Tropsch reaction. The first reaction is an endothermic reaction, whereas the second reaction is an exothermic reaction. A large amount of energy must be supplied in order to efficiently perform the first reaction, but in the second reaction, the reaction heat must be quickly removed to prevent sintering and activity deterioration of the catalyst, so it is not easy to efficiently operate the reaction process due to the characteristics of such a two-stage reaction. In particular, if the reaction heat generated during the second reaction process is not quickly removed, the reaction heat may accumulate inside the catalyst particles, causing a sintering phenomenon in which the catalyst particles agglomerate, and a deterioration problem in which the activity of the catalyst itself decreases may occur, thereby lowering the selectivity for the target hydrocarbon.

As a method for solving these problems, it is known to control the reactivity of the catalyst layer by using an inert support or inert particles together with the catalyst particles when converting carbon dioxide using a fixed bed reactor, or to use a catalyst with a new structure and characteristics. However, the former has a problem of causing side effects due to the inert support or particles used, and the newly developed catalysts do not show satisfactory results in both the conversion rate of carbon dioxide and the selectivity for high-carbon hydrocarbons at the same time.

Therefore, it is necessary to develop a novel catalyst that can produce hydrocarbons from carbon dioxide without a separate two-stage reaction process while exhibiting satisfactory results in both the conversion rate of carbon dioxide and the selectivity for high-carbon hydrocarbons.

**[Prior Art Literature]**

(Patent Document 1) KR 10-2023-0040742 A

**[Disclosure]**

**[Technical Problem]**

The present invention is intended to solve the above-mentioned problem, and aims to provide a novel carbon dioxide conversion catalyst in the form of a secondary particle formed by agglomerating a plurality of primary particles, wherein the catalyst is capable of producing high-carbon hydrocarbons, particularly hydrocarbons having 5 or more carbon atoms, with high selectivity while minimizing a sintering phenomenon at high temperatures by selectively introducing a shell layer only to the primary particles distributed on a surface area in the catalyst to produce a catalyst in the form of a core-shell, and optimizing the relationship between the diameter of the secondary core and the thickness of the shell.

**[Technical Solution]**

In order to solve the above-mentioned problem, the present invention provides a novel carbon dioxide conversion catalyst, and a carbon dioxide conversion method using the catalyst.

Specifically, (1) the present invention provides a catalyst in the form of a core-shell, wherein the core is in the form of a secondary particle formed by agglomerating a plurality of primary particles, and the shell is formed by agglomerating a plurality of primary particles in the form of a core-shell including a primary particle and a shell layer surrounding the primary particle, and wherein the average diameter (R) of the core and the average thickness (L) of the shell formed by agglomerating a plurality of primary particles in the form of a core-shell satisfy Equation 1 below:

$$[\text{Equation 1}]$$

$$0.01 \leq (L/R) \leq 0.7$$

(2) The present invention provides the catalyst according to (1) above, wherein L/R is 0.015 to 0.03.
(3) The present invention provides the catalyst according to (1) or (2) above, wherein L/R is 0.3 to 0.6.
(4) The present invention provides the catalyst according to any one of (1) to (3) above, wherein the primary particle includes Fe.
(5) The present invention provides the catalyst according to any one of (1) to (4) above, wherein the primary particle is a Fe bulk catalyst particle, or a catalyst particle in which Fe is supported as an active component on a support.
(6) The present invention provides the catalyst according to any one of (1) to (5) above, wherein the shell layer surrounding the primary particle includes Al, Ce, Cu, Co, Mo, or an oxide or nitride thereof.
(7) The present invention provides the catalyst according to any one of (1) to (6) above, wherein L is 1 to 30 $\mu$m.
(8) The present invention provides the catalyst according to any one of (1) to (7) above, wherein R is 10 to 500 $\mu$m.
(9) The present invention provides the catalyst according to any one of (1) to (8) above, wherein the catalyst is used in a reaction of producing a hydrocarbon from carbon dioxide.
(10) The present invention provides a method for converting carbon dioxide, the method including a step of synthesizing a mixed hydrocarbon gas by heating a reaction gas containing carbon dioxide in the presence of the catalyst according to any one of (1) to (9) above.

**[Advantageous Effects]**

The catalyst of the present invention has a structure in which a portion of the surface of a catalyst particle exhibiting catalytic activity is covered by a protective layer, and thus, can exhibit excellent catalytic activity while suppressing a sintering phenomenon at high temperatures, thereby stably and efficiently converting carbon dioxide into hydrocarbons, and in particular, producing a high value-added hydrocarbon having 5 or more carbon atoms among hydrocarbons with high selectivity.

**[Description of Drawing]**

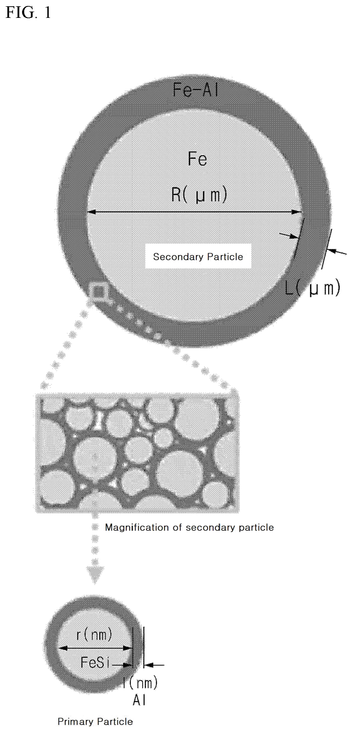

FIG. 1 is a diagram schematically illustrating the form of the catalyst of the present invention.

**[Best Modes of the Invention]**

Hereinafter, the present invention will be described in more detail.

The terms or words used in the specification and claims of the present application should not be construed as being limited to their ordinary or dictionary meanings, but should be interpreted as meanings and concepts consistent with the technical spirit of the present invention, based on the principle that the inventor may adequately define the concepts of terms to best describe his or her invention.

Catalyst for carbon dioxide conversion

The present invention provides a catalyst in the form of a core-shell, wherein the core is in the form of a secondary particle formed by agglomerating a plurality of primary particles, and the shell is formed by agglomerating a plurality of primary particles in the form of a core-shell including a primary particle and a shell layer surrounding the primary particle, and wherein the average diameter (R) of the core and the average thickness (L) of the shell formed by agglomerating a plurality of primary particles in the form of a core-shell satisfy Equation 1 below:

$$[\text{Equation 1}]$$

$$0.01 \leq (L/R) \leq 0.7$$

In the present invention, by optimizing the ratio between the diameter of the core and the thickness of the shell in a catalyst in the form of a secondary particle having a core-shell form, there is provided a novel carbon dioxide conversion catalyst which can increase the high-temperature stability of the catalyst itself while also obtaining high selectivity for high-carbon hydrocarbons having 5 or more carbon atoms, which are relatively high in added value among the finally produced hydrocarbon mixtures.

Hereinafter, the catalyst of the present invention will be described in more detail.

**Core**

The catalyst provided by the present invention has a core-shell form. The core-shell form has a structure including a core and a shell surrounding the core, and has a characteristic that the core can be protected by the shell.

Meanwhile, in the present invention, the core has a form of a secondary particle formed by agglomerating a plurality of primary particles. The primary particle constituting the core may be a Fe-based catalyst particle. The Fe-based catalyst particle refers to a catalyst particle containing Fe, which have been used in the conventional carbon dioxide conversion reaction. A reaction of converting carbon dioxide into hydrocarbons can be performed by the activity of the Fe-based catalyst particle.

More specifically, the primary particle may be a Fe bulk catalyst particle, or a catalyst particle in which Fe is supported as an active component on a support. The Fe bulk catalyst particle refers to a catalyst composed only of Fe-based components as an active component without a separate support. More specifically, Fe metal or a compound containing Fe may be used as the bulk catalyst.

Meanwhile, when the primary particle is a catalyst particle in which Fe is supported as an active component on a support, the support may be alumina or silica. The above types of supports have a large specific surface area and thus are advantageous for supporting active components, and can be particularly excellent in terms of catalytic activity and durability when Fe is supported.

Furthermore, when the primary particle is a catalyst particle in which Fe is supported as an active component on a support, a cocatalyst component may be supported together with the active component Fe for the purpose of further increasing the catalytic activity of the active component, and at least one selected from Na, K, and Cu may be used as the cocatalyst component.

Meanwhile, the core in the form of a secondary particle formed by agglomerating a plurality of primary particles may have a diameter (R) of 10 to 500 $\mu$m, preferably 10 $\mu$m or more, 15 $\mu$m or more, 20 $\mu$m or more, 25 $\mu$m or more, or 30 $\mu$m or more, and also 500 $\mu$m or less, 480 $\mu$m or less, 450 $\mu$m or less, 420 $\mu$m or less, 400 $\mu$m or less, 350 $\mu$m or less, 300 $\mu$m or less, 250 $\mu$m or less, 200 $\mu$m or less, 150 $\mu$m or less, 130 $\mu$m or less, or 100 $\mu$m or less. When the core diameter is appropriate, the catalyst can be better in terms of the mechanical strength and activity thereof.

## Shell

The shell serves to suppress catalyst sintering during the carbon dioxide conversion reaction process by covering and protecting the surface of the core described above, thereby improving the conversion rate of carbon dioxide and the selectivity for high-carbon hydrocarbons.

The shell is also formed by agglomerating a plurality of primary particles like the core described above, but the primary particle constituting the shell is characterized by having a shell layer on the primary particle itself. Since the primary particle constituting the shell have a shell layer, the aforementioned core can be protected, and the shell layer can act as a protective layer to suppress a sintering phenomenon of the catalyst.

Meanwhile, the primary particle constituting the shell may be the same as the primary particle in the core described above. Furthermore, the shell layer formed on the primary particle constituting the shell may include Al, Ce, Cu, Co, Mo, or an oxide or nitride thereof. The above components do not lower the catalytic activity of the primary particles, and have excellent high-temperature and mechanical stability, so that the performance can be sufficiently maintained even when the catalyst is used under high-temperature conditions. More preferably, the protective layer may include Al.

The shell layer may be formed through various methods, but preferably may be formed through an atomic layer deposition method. More specifically, when the protective layer is formed on the surface of the catalyst particles described above using the atomic layer deposition method, the protective layer is formed in a shape that covers only a portion of the surface of the catalyst particles while having a thin and uniform thickness. When the protective layer is formed in this shape, the conversion rate of carbon dioxide by the catalyst is improved, while the selectivity for high-carbon hydrocarbons also increases. Although the specific mechanism of this action cannot be clearly explained, it was expected that the selectivity would decrease because the surface area exhibiting activity is narrowed when the catalyst surface is covered by the protective layer, but rather, it was confirmed that the selectivity is also improved along with the conversion rate that is expected to be improved by the protective layer as in the present invention.

Meanwhile, the average diameter (R) of the core and the average thickness (L) of the shell described above are characterized by satisfying Equation 1 below:

$$[\text{Equation } 1]$$

$$0.01 \leq (L/R) \leq 0.7.$$

When the value of the (L/R), which is the ratio of the average diameter of the core and the average thickness of the shell, is within the above-described range, the carbon dioxide conversion rate and the selectivity for high-carbon hydrocarbons having 5 or more carbon atoms can be simultaneously high, and when the values of L and R are not appropriate and Equation 1 is not satisfied, at least one of the conversion rate and selectivity may be reduced.

In particular, the L/R may be 0.015 to 0.03, preferably 0.015 or more, or 0.017 or more, and 0.03 or less, 0.025 or less, or 0.2 or less. Within this range, the carbon dioxide conversion rate and the selectivity for high-carbon hydrocarbon of the catalyst can be particularly high.

In addition, in another aspect, the L/R may be 0.3 to 0.6, preferably 0.3 or more, 0.31 or more, 0.32 or more, or 0.33 or more, and 0.6 or less. Likewise, within this range, the carbon dioxide conversion rate and the selectivity for high-carbon hydrocarbon of the catalyst can be particularly high.

More specifically, the R may be 10 to 500 μm as described above, and preferably 10 μm or more, 15 μm or more, 20 μm or more, 25 μm or more, or 30 μm or more, and 500 μm or less, 480 μm or less, 450 μm or less, 420 μm or less, 400 μm or less, 350 μm or less, 300 μm or less, 250 μm or less, 200 μm or less, 150 μm or less, 130 μm or less, or 100 μm or less.

Meanwhile, the diameter (R) of the core may be adjusted by selecting the prepared secondary particles according to particle size, and the thickness (L) of the shell may be adjusted in the process of forming the primary particles having the shell layer constituting the shell. More specifically, the thickness (L) of the shell may be controlled by adjusting the amount of the shell layer precursor to be input or adjusting the flow rate in the process of forming the primary particles having the shell layer using an atomic layer deposition method. Alternatively, it may be controlled by adjusting the amount of the metal supported in the process of manufacturing the catalyst using a support method.

The L may be 1 to 30 μm, and may be 1 μm or more, or 1.5 μm or more, and 10 μm or less, 8 μm or less, 6 μm or less, 5 μm or less, 4 μm or less, or 3 μm or less, and in another case, may be 10 μm or more, 12 μm or more, 14 μm or more, 15 μm or more, or 16 μm or more, and 20 μm or less, 18 μm or less, or 17 μm or less.

Method for converting carbon dioxide

The present invention provides a method for converting carbon dioxide using the catalyst described above. More specifically, the present invention provides a method for converting carbon dioxide, the method including a step of synthesizing a mixed hydrocarbon gas by heating a reaction gas containing carbon dioxide in the presence of the above catalyst.

The selectivity for high-carbon hydrocarbons having 5 or more carbon atoms in the mixed hydrocarbon gas produced using the catalyst of the present invention may be 40% or more, preferably 45% or more, 50% or more, 55% or more, 60% or more, or 65% or more. The catalyst of the present invention is characterized in that, by having the core-shell structure described above, the conversion rate of carbon dioxide and the selectivity for high-carbon hydrocarbons are simultaneously high when performing a carbon dioxide conversion reaction using the catalyst.

The conversion reaction may be performed without any particular limitation under conditions applicable to a thermochemical conversion reaction of carbon dioxide, and the reactor used in the present invention is not particularly limited. Furthermore, the temperature at which the reaction is performed is also not particularly limited as long as it is a temperature sufficient for carbon dioxide to be converted. The catalyst of the present invention can minimize a sintering phenomenon even under high-temperature conditions by having a protective layer, so that the carbon dioxide conversion reaction can proceed stably even at relatively high temperatures.

Hereinafter, the present invention will be described in more detail by way of examples and experimental examples to specifically illustrate the present invention, but the present invention is not limited to these examples and experimental examples. However, the examples according to the present invention may be modified in various different forms, and the scope of the present invention should not be construed as being limited to the examples described in detail below. The examples of the present invention are provided to explain the present invention more completely to those skilled in the art.

**Preparation Example**

Into the precipitation reaction vessel, Fe precursor, silica as a support, and deionized water as a solvent were added. The solution was raised to 60°C while stirring at 400 rpm. Then, the aqueous precipitant solution was added to the reaction vessel at a rate of 2 ml/min using a syringe pump. When the pH reached 7 to 8, the precipitant addition was stopped, and the same temperature and stirring speed were maintained for 2 hours. Thereafter, the precipitate and the solvent were separated using a filtration device, and the precipitate was washed with deionized water, and then dried at 110°C until the precipitate was completely dry. After drying, it was calcined at 400°C for 3 hours to obtain secondary particles in which primary particles having a particle size of 10 to 20 nm were agglomerated.

Separately, Cu precursor (copper nitrate) as a cocatalyst and a K precursor (potassium nitrate) were added to deionized water to prepare a cocatalyst precursor solution, and the secondary particles were added to the cocatalyst precursor solution and mixed. After the cocatalyst component was supported, they were dried at 110°C until completely dry, and then calcined at 400°C for an additional 3 hours.

Example 1

The secondary particles obtained during the manufacturing process of the above preparation example were passed through a sieve to select secondary particles having a secondary particle size of 300 to 500 μm, and Cu and K were supported on the selected secondary particles through the process of the above preparation example. Thereafter, a certain amount of the obtained secondary particles were applied and loaded onto a square tray. Then, the tray was placed in a chamber in an ALD device, and the chamber was set to have a temperature of 150°C and a process pressure of 1 Torr. Thereafter, a process was repeated 3 times in which trimethylaluminum (TMA) at 20°C was pulse-injected into the chamber together with 100 sccm of nitrogen gas as a carrier gas for 3 seconds, maintained for 100 seconds, and then purged for 20 seconds. Thereafter, a process was repeated 3 times again in which purging was performed again for 600 seconds, and instead of trimethylaluminum in the previous process, water was pulse-injected for 3 seconds in the same manner, maintained for 100 seconds, and then purged for 20 seconds. Taking these processes as one cycle, the cycle was repeated 16 times to obtain a catalyst in the form of a core-shell.

**Example 2**

A catalyst was obtained in the same manner as in Example 1 above, except that secondary particles having a secondary particle size of 100 to 300 μm were selected and used.

**Example 3**

A catalyst was obtained in the same manner as in Example 1 above, except that secondary particles having a secondary particle size of 53 to 100 $\mu$m were selected and used.

**Example 4**

A catalyst was obtained in the same manner as in Example 1 above, except that secondary particles having a secondary particle size of 45 to 53 $\mu$m were selected and used.

**Example 5**

A catalyst was obtained in the same manner as in Example 1 above, except that secondary particles having a secondary particle size of 21 to 30 $\mu$m were selected and used.

**Example 6**

A catalyst was obtained in the same manner as in Example 3 above, except that the number of cycle repetitions was adjusted to 1 time.

**Example** 7

The secondary particles obtained during the manufacturing process of the above preparation example were passed through a sieve to select secondary particles having a secondary particle size of 100 to 300 $\mu$m, and a certain amount of the selected secondary particles were mixed with a precursor solution in which copper nitrate, potassium nitrate, and aluminum nitrate were dissolved. The concentration of the aluminum precursor in the mixture was adjusted to 40 wt%, and the secondary particles and the precursor solution were mixed so that the weight of the aluminum element in the final catalyst was 10 wt%. The mixture was stirred at room temperature, dried at 100°C for 8 hours, and then calcined at 400°C for 3 hours to obtain a catalyst.

**Example 8**

A catalyst was obtained in the same manner as in Example 7 above, except that the weight of the aluminum element in the final catalyst was adjusted to 20 wt%.

**Comparative Example 1**

The secondary particles obtained in Preparation Example 1 above were used as a catalyst as they are.

**Comparative Example 2**

A catalyst was obtained in the same manner as in Example 7 above, except that the weight of the aluminum element in the final catalyst was adjusted to 40 wt%.

**Comparative Example 3**

A catalyst was obtained in the same manner as in Example 7 above, except that the weight of the aluminum element in the final catalyst was adjusted to 50 wt%.

When the secondary particle diameter (core diameter) of the prepared catalyst is R, and the thickness of the shell is L, each of these values can be measured and calculated by the following method.

- Method for measuring L and R values

The prepared catalyst is epoxy molded and cut to obtain a cross-section, and the cross-section is analyzed by SEM and EDS to obtain an image.

More specifically, a clear element distribution image is obtained by long-term exposure of 4 hours or more using EDS mapping analysis, and the Al and Fe element distribution images are defined by drawing the cross-sectional boundaries of Fe and Al as closed curves based on the density-based spatial clustering of applications with noise (DBSCAN) algorithm for the obtained image. In this case, the distribution of the Fe element may be atypical, have holes, and have a molded body in addition to the Fe element inside.

The atypical figure, which is the form of the Fe element distribution, is divided into very small polygons, and in this process, a part with a hole and a molded body part are excluded. When the center of mass of each polygon is $(x_i, y_i)$ and the area of each polygon is $A_i$, the values of the products of the mass center and the area of each polygon are added up, and then divided by the total area to obtain the mass center $(C_x, C_y)$ of the entire figure. Meanwhile, the coordinates of the mass center of each polygon are the average of the vertex coordinates of each figure.

$$C_x = \frac{\sum (x_i \cdot A_i)}{\sum A_i} , \quad C_y = \frac{\sum (y_i \cdot A_i)}{\sum A_i} ,$$

Meanwhile, the R value can be calculated from the A value obtained by summing all the area values $A_i$ of each of the polygons using the following equation:

$$R = 2(A/\pi)^{1/2}$$

In addition, the cross-sectional boundary of Al is similarly defined by drawing it as a closed curve, and the atypical figure, which is the Al element distribution, is divided into very small polygons, and in this process, a part with a hole and a molded body part are excluded. When the area value of each of the small polygons is $B_i$, the total area B of the shell can be calculated as the sum of the $B_i$ values.

In addition, the distance between the previously obtained $(C_x, C_y)$ and the center of each polygonal figure divided in the shell is calculated, and the average thereof is defined as $L_{start}$. In this case, the thickness L of the shell satisfies the B and $L_{start}$ obtained previously and the following equation, and the L value can be calculated using the following equation:

$$B = \pi((L_{start} + L)^2 - (L_{start})^2)$$

Through the above method, the R and L values for the catalysts of the examples and comparative examples were measured, and the L/R value was calculated. The resulting values are summarized in Table 1 below.

[Table 1]

|  | L value ($\mu$m) | R value ($\mu$m) | L/R |
|---|---|---|---|
| Example 1 | 16 | 480 | 0.033 |
| Example 2 | 12 | 215 | 0.056 |
| Example 3 | 17 | 78 | 0.218 |
| Example 4 | 16 | 48 | 0.333 |
| Example 5 | 17 | 34 | 0.500 |
| Example 6 | 1.5 | 82 | 0.018 |
| Example 7 | 35 | 78 | 0.449 |
| Example 8 | 40 | 70 | 0.571 |
| Comparative Example 1 | 0 | 425 | 0 |
| Comparative Example 2 | 61 | 78 | 0.782 |
| Comparative Example 3 | 85 | 98 | 0.867 |

**Experimental Example 1. Measurement of conversion rate and selectivity in carbon dioxide conversion reaction using catalyst**

Mixed hydrocarbons were prepared from carbon dioxide using the catalysts prepared in the above examples and comparative examples. Specifically, 0.5 g of the catalyst was loaded into a fixed-bed tubular reactor (Stainless steel, diameter 1/2 inch), and the catalyst was activated by reduction treatment for 8 hours under a carbon monoxide atmosphere (>99.9 volume%) at 400°C. Thereafter, a mixed gas mixed at a volume ratio of $H_2:CO_2:N_2=54:18:3$ was flowed at a flow rate of 75 ml/min to perform the reaction at 340°C and 20 bar conditions.

The reaction product formed as a result of the reaction was passed through a constant temperature water bath trap set to 0°C to collect liquid hydrocarbons having 5 or more carbon atoms, and the gaseous product was analyzed in real time using gas chromatography. The reaction was performed continuously for 48 hours or more.

The carbon dioxide conversion rate and the selectivity for hydrocarbons having 5 or more carbon atoms were measured/calculated using the following methods.

1) Conversion rate: calculated using the following equation:

Carbon dioxide conversion rate = {(input $CO_2$ flow rate - discharge $CO_2$ flow rate) / (input $CO_2$ flow rate)} * 100%

2) Selectivity: calculated using the following equation:

Selectivity for gaseous products = {($C_AH_B$ flow rate * A) / (input $CO_2$ flow rate - discharge $CO_2$ flow rate)} * 100%

Selectivity for liquid hydrocarbons with 5 or more carbon atoms = 100% - (total selectivity for gaseous products)

The measured conversion rate and selectivity values are summarized in Table 2 below.

[Table 2]

| | CO$_2$ Conversion rate (%) | C5+ selectivity (%) |
|---|---|---|
| Example 1 | 52.5 | 60.1 |
| Example 2 | 50.6 | 67.9 |
| Example 3 | 50.6 | 67.9 |
| Example 4 | 56 | 68 |
| Example 5 | 55.9 | 68.1 |
| Example 6 | 57.8 | 69.9 |
| Example 7 | 55.4 | 67.1 |
| Example 8 | 54.5 | 65.2 |
| Comparative Example 1 | 44.4 | 46.5 |
| Comparative Example 2 | 47.7 | 55.5 |
| Comparative Example 3 | 46.4 | 54.4 |

As can be seen from Table 2 above, it was confirmed that when using the catalysts of the examples of the present invention, the carbon dioxide conversion rate and the selectivity for liquid hydrocarbons with 5 or more carbon atoms having high added value were higher than when using the catalysts of the comparative examples whose value of L/R is outside the range of the present invention.

**Claims**

1. A catalyst in the form of a core-shell,

   wherein the core is in the form of a secondary particle formed by agglomerating a plurality of primary particles, and the shell is formed by agglomerating a plurality of primary particles in the form of a core-shell including a primary particle and a shell layer surrounding the primary particle, and
   wherein the average diameter (R) of the core and the average thickness (L) of the shell formed by agglomerating a plurality of primary particles in the form of a core-shell satisfy Equation 1 below:

[Equation 1]

$$0.01 \leq (L/R) \leq 0.7$$

2. The catalyst according to claim 1, wherein L/R is 0.015 to 0.03.

3. The catalyst according to claim 1, wherein L/R is 0.3 to 0.6.

4. The catalyst according to claim 1, wherein the primary particle includes Fe.

5. The catalyst according to claim 1, wherein the primary particle is a Fe bulk catalyst particle, or a catalyst particle in which Fe is supported as an active component on a support.

6. The catalyst according to claim 1, wherein the shell layer surrounding the primary particle includes Al, Ce, Cu, Co, Mo, or an oxide or nitride thereof.

7. The catalyst according to claim 1, wherein L is 1 to 30 $\mu$m.

8. The catalyst according to claim 1, wherein R is 10 to 500 $\mu$m.

9. The catalyst according to claim 1, wherein the catalyst is used in a reaction of producing a hydrocarbon from carbon dioxide.

10. A method for converting carbon dioxide, the method including a step of synthesizing a mixed hydrocarbon gas by heating a reaction gas containing carbon dioxide in the presence of the catalyst of claim 1.

FIG. 1

## INTERNATIONAL SEARCH REPORT

| International application No. |
|---|
| **PCT/KR2024/014045** |

### A. CLASSIFICATION OF SUBJECT MATTER

**B01J 35/30**(2024.01)i; **B01J 35/40**(2024.01)i; **B01J 23/745**(2006.01)i; **C07C 1/12**(2006.01)i; **C10G 2/00**(2006.01)i

According to International Patent Classification (IPC) or to both national classification and IPC

### B. FIELDS SEARCHED

Minimum documentation searched (classification system followed by classification symbols)

B01J 35/30(2024.01); B01J 29/40(2006.01); B01J 29/46(2006.01); B01J 31/16(2006.01); B01J 35/00(2006.01); B01J 37/00(2006.01); C01B 32/40(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 촉매(catalyst), 이산화탄소(carbon dioxide), 전환(conversion), 코어-쉘(core-shell), 1차 입자(primary particle), 2차 입자(secondary particle)

### C. DOCUMENTS CONSIDERED TO BE RELEVANT

| Category* | Citation of document, with indication, where appropriate, of the relevant passages | Relevant to claim No. |
|---|---|---|
| A | WEBER, D. et al. Carbon nanosphere-encapsulated fe core–shell structures for catalytic co2 hydrogenation. ACS Applied Nano Materials. 2022, vol. 5, no. 8, pp. 11605-11616. See abstract; pages 11606-11610; and formula 2. | 1-10 |
| A | KR 10-2023-0129077 A (RESEARCH & BUSINESS FOUNDATION SUNGKYUNKWAN UNIVERSITY) 06 September 2023 (2023-09-06) See claims 1-22. | 1-10 |
| A | CN 109985659 A (NANJING INSTITUTE OF TECHNOLOGY) 09 July 2019 (2019-07-09) See claims 1-8. | 1-10 |
| A | KR 10-2020-0091656 A (KOREA RESEARCH INSTITUTE OF CHEMICAL TECHNOLOGY) 31 July 2020 (2020-07-31) See claims 1-12. | 1-10 |

☑ Further documents are listed in the continuation of Box C.    ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **19 December 2024** | **19 December 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2024/014045** |

### C.    DOCUMENTS CONSIDERED TO BE RELEVANT

| Category* | Citation of document, with indication, where appropriate, of the relevant passages | Relevant to claim No. |
|---|---|---|
| A | KR 10-2023-0066207 A (KOREA ADVANCED INSTITUTE OF SCIENCE AND TECHNOLOGY) 15 May 2023 (2023-05-15)<br>    See claims 1-25. | 1-10 |

Form PCT/ISA/210 (second sheet) (July 2022)

**INTERNATIONAL SEARCH REPORT**
Information on patent family members

| Patent document cited in search report | | | Publication date (day/month/year) | Patent family member(s) | | | Publication date (day/month/year) |
|---|---|---|---|---|---|---|---|
| KR | 10-2023-0129077 | A | 06 September 2023 | KR | 10-2700864 | B1 | 30 August 2024 |
| | | | | WO | 2023-163573 | A1 | 31 August 2023 |
| CN | 109985659 | A | 09 July 2019 | CN | 109985659 | B | 01 March 2022 |
| KR | 10-2020-0091656 | A | 31 July 2020 | KR | 10-2168444 | B1 | 21 October 2020 |
| | | | | US | 11865521 | B2 | 09 January 2024 |
| | | | | US | 2022-0088578 | A1 | 24 March 2022 |
| | | | | WO | 2020-153780 | A1 | 30 July 2020 |
| KR | 10-2023-0066207 | A | 15 May 2023 | KR | 10-2611089 | B1 | 08 December 2023 |
| | | | | KR | 10-2611089 | B9 | 13 March 2024 |

Form PCT/ISA/210 (patent family annex) (July 2022)

**EP 4 778 629 A1**

**Patent documents cited in the description**

- KR 1020230123492
- KR 1020230040742 A